# EUROPEAN PATENT APPLICATION

(11) **EP 4 628 011 A2**
(43) Date of publication of application: **08.10.2025**
(21) Application number: 25182589.9
(22) Date of filing: 26.10.2020
(51) Int. Cl.: A61B 5/12

(54) **A METHOD FOR DETECTING A PLURALITY OF HEALTH CONDITIONS OF A COCHLEA**

(30) Priority: 07.11.2019 EP 19207745
(62) Divisional of application: 22158539.1
(71) Applicant: Cochlear Limited, Macquarie University, New South Wales 2109 (AU)
(72) Inventor: STAHL, Pierre, 06220 Vallauris (FR); FRATER, Attila, London, WC1E 6BT (GB); SEGOVIA MARTINEZ, Manuel, 06220 Vallauris (FR); GNANSIA, Dan, 06220 Vallauris (FR); MOLAEE, Behnam, 06220 Vallauris (FR); DEMARCY, Thomas, 06560 Valbonne (FR)
(74) Representative: Grünecker Patent- und Rechtsanwälte PartG mbB

(57) **Abstract**

A system, comprising: a transducer configured to apply acoustic stimulation to a recipient of a cochlear implant; an electrode array that is configured for insertion into at least a portion of a recipient's cochlea; and a processor unit coupled to the electrode array, wherein the system is configured to provide a personalized and automatic fitting of a cochlea implant frequency allocation map based on based on eCOchG amplitude responses measured by one or more of the electrodes of the electrode array.

## Description

### FIELD

The present disclosure relates to hearing aid devices. More particularly, the disclosure relates to a method for detecting a plurality of health conditions of a cochlea.

### BACKGROUND

It is well known that Cochlea Implant (CI) surgery, mainly during the electrode array insertion, can induce traumas into the cochlea. Scientific and medical society in the CI field have attempted to minimize the risks of insertion traumas by improving electrode array designs, surgical procedures and anatomic knowledge.

An objective measure known as electrocochleography (eCochG) has shown to lower the risks of traumatic surgeries. One of the eCochG component is Cochlea Microphonics (CM), i.e. the response of hair cells to acoustic tone bursts. Usually, an eCochG is recorded from acoustical sine and cosine tone bursts (named rarefaction and condensation stimuli) that are generated sequentially or alternatingly. The CM is one of the components of the eCochG, and is easily detected from their response in phase with the stimulation.

The eCochG can be recorded either with: intracochlear electrodes (from electrodes of an electrode array of a cochlea implant during the electrode insertion), or with extracochlear electrodes (surface electrodes arranged on an outer wall of a cochlea combined either with an external recording system or embedded in the CI as a recording, floating external electrode - e.g. the reference electrode of the CI).

It has been proven that CMs, recorded by eCochG systems, can provide information to the surgeon about cochlea health conditions during the CI surgery. During insertion of the electrode array, eCochGs are constantly being recorded by a recording electrode being the most apical electrode of the electrode array (intracochlear eCochG) in response to acoustical bursts at one frequency, preferably 500 Hz. By inserting the electrode array, the recording electrode gets closer to the centre of excitation of hair cells (tonotopically situated at the cochlea apex). The CMs are then supposedly to increase as long as the electrode array is inserted, until reaching a plateau of saturation. In the case were the electrode is damaging the cochlea (blocking the basilar membrane from vibration, translocated to the scala vestibuli, piercing the cochlea wall, etc ...), the CMs suddenly drops. It has been proven that the residual hearing after the cochlea implant surgery of the patient is extremely correlated to these suddenly drops in the CMs.

eCochG monitoring CI surgery are then known for contributing to atraumatic CI insertion.

However, one potential drawback of the described method is that no feedback is given to the surgeon about the region of the cochlea damaged by the electrode insertion and the depth of electrode insertion when a damage occurs. It can then be complex for the surgeon to estimate the potential trauma made by the electrode array when a drop of eCochG occurs, i.e. drop in the measure of CM.

### SUMMARY

An aspect of the disclosure is to provide a method for detecting a health condition of a cochlea during insertion of an electrode array of a cochlea implant system.

A further aspect of the disclosure is to provide a method which improves the surgery's ability to insert an electrode array of a cochlea implant system with minimum reduction of the residual hearing of the user after the insertion of the cochlea array.

An even further aspect of the disclosure is to provide a personalized and automatic fitting of the cochlea implant frequency allocation map based on the patient response, after the surgery.

The aspect of the disclosure is achieved by a method for detecting a plurality of health conditions of a cochlea during insertion of an electrode array of a cochlear implant system into the cochlea of a recipient. The method comprising applying a plurality of acoustic stimulations, and where each of the plurality of acoustic stimulations includes a tone burst with different frequencies. The plurality of acoustic stimulations may be applied by a receiver which could be a transducer placed at or in an ear canal of the user or a vibrator configured to apply a mechanical vibration onto a temporal bone of the user. The tone burst includes multiple acoustic pulses modulated at a tone of one frequency selected from a range of frequencies which is audible. An electrocochleography probe is arranged onto an outer wall of the recipient's cochlea for measuring a plurality of cochlea baseline responses based on the plurality of acoustic stimulations, before insertion of the electrode array into the recipient's cochlea.

For each of the plurality of acoustic stimulations the electrocochleography probe measures a cochlea baseline response before the insertion of the electrode array into the recipient's cochlea. The cochlea baseline response includes cochlea response based on an acoustic stimulation before any potential trauma is applied to the cochlea due to insertion of the electrode array. Then, during insertion of the electrode array into the recipient's cochlea, the electrocochleography probe measures continuously a plurality of cochlea responses based on the plurality of acoustic stimulations. A plurality of electrical response differences between the plurality of cochlea baseline responses and the plurality of cochlea responses is determined. For each of the plurality of electrical response differences includes a determined difference between a cochlea baseline response of the plurality of cochlea baseline responses and a cochlea response of the plurality of cochlea responses, where the cochlea baseline response and the cochlea response are both measured based on an acoustic stimulation including the same tone burst. A plurality of health conditions of the cochlea is determined during insertion of the electrode array based on the plurality of electrical response differences.

The acoustic stimulation could be provided to the recipient of the electrode array via a speaker or a bone anchored hearing system.

The electrocochleography probe may be an extracochlear probe. The advantage of using an extracochlear probe rather than using the electrodes of the electrode array as the electrocochleography probe, such as in an intracochlear system, is that the stability of recording cochlea responses is improved and the user-friendly of the extracochlear probe setup is also improved. The reason for it is that the Signal-To-Noise ratio (SNR) of the recording of cochlea response of the intracochlear probe setup varies because of the movement of the electrode array during the insertion of the electrode array. In practically, a drop in the SNR is seen during the insertion of the electrode while using one or more electrodes as recording electrodes of the cochlea response, and the drop in the SNR will result in inaccurate determination of the health condition of the cochlea and position of the electrode array in the cochlea. However, the stability and user-friendly of the intracochlear probe setup is optimized if applying sequentially an electrical stimulation to each of the plurality of electrodes of the electrical array, performing a plurality of impedance measurements of each plurality of electrodes during insertion of the electrode array into the recipient's cochlea based on the electrical stimulations, and determine a plurality of correlation signals based on the plurality of impedance measurements and the plurality of electrical response differences, respectively. The impedance measurements will be significantly helpful in determine the health condition and position of the electrode array in a situation when the SNR drops.

Alternatively, the plurality of cochlea baseline response may be statistical determined based on previous measured or recorded cochlea baseline responses from either the recipient of the electrode array or other users of cochlea implant.

The plurality of health conditions of the cochlea is determined or assessed at different location on the cochlea by changing the frequency of the tone burst.

The plurality of cochlea baseline responses may correspond to a plurality of baseline eCochG measures and the plurality of cochlea responses corresponds to a plurality of eCochG measures, and each of the plurality of electrical response corresponds to a subtraction between a baseline eCochG measure with a corresponding eCochG measure during electrode insertion.

Each of the plurality of health conditions may include determination of eCochG or an eCochG trauma index, where the eCochG trauma index indicates the amount of reduction in measured cochlea microphonics due to a collision between the electrode array being inserted and the inner wall of the cochlea.

A further aspect of the disclosure is achieved by a system comprising, a receiver configured to apply a plurality of acoustic stimulations to a recipient, and where each of the plurality of acoustic stimulations includes a tone burst with different frequencies. Furthermore, the system includes a cochlea implant system comprising an electrode array configured to provide an electrical stimulation to auditory nerves of the recipient's cochlea, and the electrode array includes a plurality of electrodes. Additionally, the system includes a stimulator processor configured to apply the electrical stimulation to one or more of the plurality of electrodes. The stimulator processor may be part of the cochlea implant system or part of an external stimulator connected to a processor unit.

The system comprises an electrocochleography probe arranged on to an outer wall of the recipient's cochlea, and where the electrocochleography is configured to perform measurements of a plurality of cochlea baseline responses based on the plurality of acoustic stimulations, before insertion of the electrode array into the recipient's cochlea, and to perform continuously or sequentially measurements of a plurality of cochlea responses based on the plurality of acoustic stimulations, during insertion of the electrode array into the recipient's cochlea.

The electrocochleography probe is a medical device which includes a sensor for measuring an eCochG response.

The system further comprises a processor unit connected at least to the electrocochleography probe, and where the processor is configured to determine a plurality of electrical response differences between the plurality of cochlea baseline responses and the plurality of cochlea responses, respectively, and to determine a plurality of health conditions within the cochlea at different locations during insertion of the electrode array based on the plurality of electrical response differences.

The processor unit may be part of a computer which is connected to the cochlea implant system via a wired or a wireless interface, where the communication between the computer and the cochlear implant system comprises the plurality of cochlea baseline responses and the plurality of cochlea responses. The processor unit is then configured to transfer the determined plurality of health conditions to a graphical user interface of the computer. Alternatively, the processor unit may be part of a tablet, a smart phone or any kind of a computer device.

The advantage of the method and the system is that the surgery is able to monitor the health of the cochlea while inserting the electrode array into the cochlea. In a situation where each of the plurality of acoustic stimulations are continuously being applied to the user acoustically, the surgery can monitor the health conditions of the cochlea and the location of the trauma on the cochlea. For example, the recipient receives a tone burst at 250 Hz and at 1000 Hz, and if the surgery sees a drop in the eCochG measure, i.e. the determined electrical response difference becomes negative, at one or both of the two frequencies, then it is possible for the surgery to locate whether the trauma has appeared in the apex region and/or more basilar region of the cochlea.

The precision in determining the location of the trauma is depending on the number of acoustic stimulations of the plurality of acoustic stimulations. A higher number of acoustic stimulations provides a better resolution in determining the location of the trauma, that means, the precision of the determined location of the trauma has improved.

The plurality of baseline responses and the plurality of cochlea responses include measurements of cochlea microphonics of the cochlea.

The cochlear microphonic (CM) is an alternating current (AC) voltage that mirrors the waveform of the acoustic stimulus from the cochlea.

The acoustic stimulation is provided by a receiver configured to provide the plurality of stimulations that evoke basilar membrane vibrations. The plurality of stimulations may originate from an ear canal acoustical stimulator (via a speaker driven from a computer or hearing aid device), middle ear implant stimulator configured to provide vibration support of the ossicles in the middle ear of the recipient or bone conduction device configured to provide the plurality of acoustic stimulations via a temporal bone of the recipient.

The electrical array may include a plurality of electrodes, and wherein the method further comprising applying sequentially an electrical stimulation to each of a plurality of electrodes of the electrical array, performing or recording a plurality of impedance measurements of each plurality of electrodes during insertion of the electrode array into the recipient's cochlea based on the electrical stimulations, and determining a plurality of correlation signals based on the plurality of impedance measurements and the plurality of electrical response differences.

An impedance measurement of an electrode is used for detection of whether the respective electrode of which the impedance is measured is within or outside the cochlea. When the electrode of the electrode array is outside the cochlea the impedance is not measurable, i.e. the impedance is infinite. When the electrode of the electrode array is within the cochlea, the impedance is measurable, i.e. the impedance is finite.

The plurality of cochlea baseline response may be recorded by the electrocochleography probe before the insertion of the electrode array into the recipient's cochlea, but, during or after insertion of the electrode array the position of the probe can be shifted on the cochlea while adapting the plurality of cochlea baseline response based on the plurality of impedance measurements to the new position of the probe. For example, when changing the position of the probe on the cochlea from a first position to a second position, the electrode array will see a change in the recorded plurality of impedance measurements from when the probe was placed at the first position to the time the probe was placed at the second position. The change in the plurality of impedance measurements can be used for adapting the plurality of cochlea baseline response to the new position of the probe.

Each of the plurality of correlation signal is determined by a relation between an impedance measurement of the plurality of impedance measurements and the plurality of electrical response differences.

By the plurality of impedance measurements, it is possible to determine the location of the electrode array within the cochlea, and by correlating the plurality of impedance measurements and the plurality of electrical response differences, a reason of the change in health condition is possible to be determined. For example, if the location of the electrode array is determined to be within the basilar region of the cochlea and the location of the trauma is determined based on the plurality of electrical response differences to be in the apex region of the cochlea, the reason for the trauma may be due to an error in the electrocochleography probe because the electrode array has not yet entered the basilar region. Thus, if the location of the electrode array is determined to be within the apex region of the cochlea and the location of the trauma is in the apex region of the cochlea, the reason for the trauma may be due to a collision between the electrode array and the wall of the cochlea, and more specifically, between a tip of the electrode array and the wall of the cochlea. Thus, if the location of the electrode array is determined to be within the apex region of the cochlea and the location of the trauma is in the basilar region of the cochlea, the reason for the trauma may be due to a collision between a part of the electrode array which is not the tip of the electrode array and the wall of the cochlea or an error in the electrocochleography probe.

The plurality of correlation signals may be further determined based on a length of the electrode array, a length of each of the plurality of electrodes, and/or a distance between each of the plurality of electrodes. Thereby, it is possible to determine the location of the electrode array within the cochlea even more precise.

The plurality of impedance measurements comprises determining real-time and/or continuously impedance measurements of each of the plurality of electrodes.

By combining the real-time and/or continuously impedance measurements and the real-time and/or continuously plurality of health conditions the surgery is able to stop the insertion of the electrode array when a trauma is detected. Thereby, the damaging of the residual hearing of the recipient is reduced in comparison to the situation where the surgery is not aware of the trauma and continuing the insertion of the electrode array.

The electrode array includes a plurality of electrodes, such as at least 15 electrodes.

The stimulator processor is configured to apply sequentially an electrical stimulation to each of the plurality of electrodes of the electrical array, and based on the electrical stimulations, the stimulator processor is configured to perform a plurality of impedance measurements of each plurality of electrodes during insertion of the electrode array into the recipient's cochlea. The stimulator processor is then configured to determine a plurality of correlation signals based on the plurality of impedance measurements and the plurality of electrical response differences, respectively.

A first state and a second state for each of the electrodes of the electrode array may be determined based on each of the plurality of impedance measurements, and wherein an electrode of the electrode array is in the first state if the impedance measurement is above a first impedance level, and the electrode is in the second state if the impedance measurement is below the first impedance level.

The electrode of the electrode array is located outside the cochlea if the electrode is in the first state, and the electrode of the plurality of electrodes is located within the cochlea if the electrode is in the second state. In first state the measured impedance is infinite, i.e. no electrical conduction is measurable. In the second state the measured impedance is finite, i.e. electrical conduction is measurable.

The plurality of health conditions includes a first health condition and a second health condition, and where the cochlea is in the first health condition if a respective electrical response difference of the plurality of electrical response difference is larger than a first response difference threshold, and where the cochlea is in the second health condition if the respective electrical response difference of the plurality of electrical response difference is below a first response difference threshold.

The plurality of health conditions includes at least a first health condition and at least a second health condition, and where the cochlea is in the at least first health condition if an electrical response difference of the plurality of electrical response difference is above or equal a response difference threshold, and where the cochlea is in the at least second health condition if the respective electrical response difference of the plurality of electrical response difference is below the response difference threshold.

The response difference threshold is determined based on a cochlea baseline response of the plurality of cochlea baseline responses, where the determined electrical response difference and the response difference threshold are determined based on the same acoustic stimulation. Thereby, the cochlea baseline response of the plurality of cochlea, the cochlea response of the plurality of cochlea response, the electrical response difference of the plurality of electrical response difference and the response threshold are all related to the same acoustic stimulation.

The determining of the plurality of health conditions comprises determining real-time or continuously plurality of health conditions.

The system may include a graphical user interface, where the graphical user interface is configured to provide a graphical representation of the plurality of electrical response differences as a function of insertion time, physiological cochlea place-frequencies, and/or insertion depths.

The system may include an alarm unit which is configured to generate an alarm signal if the first electrical response difference and/or the second electrical response difference is below a first response difference threshold, i.e. the health of the cochlea is in a second health condition. The alarm unit may be part of the computer including the processor unit. The alarm signal may be transmitted to the graphical user interface, or the alarm signal may be transferred to the surgery as an acoustic alarm sound. The graphical user interface may be configured to convert the alarm signal into a warning message or warning sound which is used by the user of the graphical user interface to stop the insertion of the electrode array or to turn the electrode array.

The graphical user interface may be configured to provide a graphical representation of the plurality of correlation signals as a function of physiological cochlea place-frequencies and/or insertion depths.

The system may include a diagnose unit which is configured to generate a message which includes the reason of the alarm signal and/or a status of each of the electrode of the electrode array. The diagnose unit is connected to the processor unit and/or the alarm unit.

The system may include a memory wherein a physiological cochlea place-frequency map, derived from for example Greenwood (1961), is stored. The graphical user interface is connected to the memory. The physiological cochlea place-frequency map may be customized to the recipient of the cochlea implant system by the use of a 3D image of the recipient's cochlea.

The tone burst may include a sweep tone which is a continuous acoustic signal in which the frequency varies over time. A sweep tone may cover the full audible frequency spectrum (20 - 20 000Hz) but could be used in a more limited frequency spectrum, such as two or more tones generated in a continuous manner.

The tone burst may include a harmonic complex acoustic signal composed of several tones, e.g. two or more pure tones. The composition of the tones may either be a convolution or a multiplication of tones.

The shape of the sweep tone or the harmonic complex acoustic signal may be adapted to an electrocochleography profile which provides the length in time, amplitude of each tone, and the timing of when each of the tone begins. For example, within a first time window having a first time length, the amplitude of one or more tones increases gradually over time, and within a second time window having a second time length, the amplitude of the one or more tones are held constant over time, and within a third time window having a third time length, the amplitude of the one or more tones are reduced gradually over time. The second time window is subsequent to the first time window, and the third time window is subsequent to the second time window.

The electrocochleography profile may be stored in the memory of the system.

The electrocochleography profile may have at least two time windows with either different or same time length, and in a first time window of the at least two time windows one or more tones changes gradually over time with a first rate, and in a second time window of the at least two time windows one or more tones changes gradually over time with a second rate, wherein the first rate is different from the second rate.

The position of the electrocochleography probe can be determined based on the tone burst including different frequencies, i.e. different tones. A cochlea response is measured by the probe for each of the frequencies, and a maximum cochlea response is determined among the measured cochlea responses for each of the frequencies. The cochlea responses may be part of the plurality of cochlea responses. The maximum cochlea response has the highest response amplitude of the plurality of cochlea responses. The position of the electrocochleography probe can be determined by extracting the position from the physiological cochlea place-frequency map based on the frequency which provides the maximum cochlea response. The position of the probe may be determined by the processor unit.

The physiological cochlea place-frequency map may be stored in the memory of the system.

In a tone burst the multiple frequencies arrives to the cochlea at different time. For example, a most basal tone of the tone burst comes after a most apical tone of the tone burst, and the different time shifts between the different tones are known and stored in the memory of the system. The electrocochleography probe measures a plurality of cochlea responses based on the tone burst, and a time shift between the plurality of cochlea responses is determined by the processor unit. Based on the time shifts the processing unit is configured to determine whether the residual hearing of the cochlea has become worse during and/or after the insertion of the electrode array. For example, if the time shift has increased then the residual hearing of the cochlea has decreased.

After the insertion of the electrode array, a frequency map of the electrodes of the electrode array may be determined. The frequency map includes optimal stimulation frequencies for each of the electrodes. The frequency map may be determined by a sound processor of a cochlear implant system, where the sound processor is connected to the electrode array, or by the processor unit of the system where the processor unit is connected to the electrode array via the sound processor of the cochlea implant system or via an implant of the cochlea implant system. First, a baseline frequency and a baseline cochlea response of a given electrode of the electrode array must be found. This is achieved by applying a plurality of acoustic stimulations to the user of a cochlea implant system including the inserted electrode array, and where each of the plurality of acoustic stimulations includes a tone or a tone burst with different frequencies, e.g. 300 Hz, 350 Hz, 400 Hz, 450 Hz, 500 Hz, 550 Hz, and 600 Hz. Then, a cochlea response, eCOchG amplitude response, is measured by one or more of the electrodes of the electrode array and for each of the different frequencies. For example, the frequency of the different frequencies which provides the highest cochlea response, i.e. the highest eCochG amplitude response, is the baseline frequency, and the highest cochlea response is the baseline cochlea response. The one electrode is preferably the most apical electrode of the electrodes of the electrode array. For example, the baseline frequency response is 400 Hz and the one electrode is the most apical electrode, i.e. electrode number 20.

The baseline frequency and the baseline cochlea response for the one electrode may be stored in the memory unit of the system or within a memory of the cochlea implant system.

The one electrode is denoted as the baseline electrode.

Next, a single acoustic stimulation is applied to the user, wherein the single acoustic stimulation includes the baseline frequency, e.g. 400 Hz. A cochlea response is measured by a group of electrodes which includes electrodes of the electrode array other than the baseline electrode, e.g. other than electrode number 20. For example, the group of electrodes includes electrode number 13, 10, 7 and 3.

A cochlea response model including a cochlea response distribution along a cochlea to a given tone may be derived based on collected data, a general shape of a cochlea response distribution profile along a cochlea, surface image or 3D field image of the cochlea.

The cochlea response distribution of the cochlea response model includes a maxima cochlea response, and the maxima cochlea response is centered at the baseline frequency, and the maxima cochlea response is equal to the baseline cochlea response. The measured cochlea response for each of the electrode of the group of electrodes, e.g. electrode number 13, 10, 7 and 3, is matched to a hypothesized frequency where the measured cochlea response is equal to a theoretical cochlea response of the cochlea response model.

Each of the electrode of the group of electrodes is matched to a hypothesized frequency.

Thereby, the frequency map of the electrode array may include the baseline frequency of the baseline electrode and the hypothesized frequencies of the group of electrodes.

The frequency map of the electrode array may be derived by applying a curve fitting method to the baseline frequency of the baseline electrode and the hypothesized frequencies of the group of electrodes for determine a hypothesized frequency to each of the electrodes of the electrode array or the remaining electrodes of the electrode array.

The electrocochleography probe arranged on to an outer wall may be replaced by using one or more of the plurality of electrodes of the electrode array as recording electrodes for performing measurements of a plurality of cochlea baseline responses based on the plurality of acoustic stimulations on each of the one or more electrodes before insertion of the electrode array into the recipient's cochlea, and performing continuously measurements of a plurality of cochlea responses based on the plurality of acoustic stimulations, during insertion of the electrode array into the recipient's cochlea,

Knowing the disadvantages of using an intracochlear probe setup in comparison to an extracochlear probe setup, it is of advantage to combine the intracochlear probe setup with impedance measurements for improving the precision in determine the plurality of health conditions and location when experience a drop in the SNR of the cochlea response measure provided by the electrode array.

For reducing the friction force when inserting the electrode array, and thereby, for reducing the risc of damaging the residual hearing when the electrode array touches the inner wall of the cochlea, a vibrator may be placed on the same side of the head as where the electrode array is to be inserted or on the opposite side of head. During insertion of the electrode array, the vibrator evokes acoustic soundwaves into the skull of the recipient of the electrode array, and the acoustic soundwaves will reduce the friction force while inserting the electrode array. For the acoustic soundwave not to interfere with the measurements of the cochlea responses, the frequencies(y) of the acoustic soundwaves are selected to be different from the frequency of the tone burst.

The vibrator may be arranged on an abutment which is attached to a screw arranged into the skull. Alternatively, the vibrator may be attached on the head via a magnetic attraction to an implant. Alternatively, the vibrator may be attached on the head via a softband arranged on the head.

### BRIEF DESCRIPTION OF DRAWINGS

The aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effect will be apparent from and elucidated with reference to the illustrations described hereinafter in which:
- Fig. 1: illustrates a diagram of a method,
- Figs. 2A and 2B: illustrate different configuration of a system,
- Fig. 3: illustrates a diagram of the method,
- Figs. 4A to 4F: illustrate the method,
- Figs. 5A to 5C: illustrate different examples of a situation during insertion of the electrode array,
- Figs. 6A and 6B: illustrate the method and the system,
- Fig. 7: illustrates the method and the system,
- Figs. 8A and 8B: illustrate a graphical user interface,
- Figs. 9A and 9B: illustrate different examples of a plurality of acoustic stimulations,
- Figs. 10A and 10B: illustrate the tone burst,
- Figs 11A and 11B: illustrate cochlear response based on one or more tone bursts,
- Figs. 12A to 12D: illustrate an example of determining a frequency map of the electrodes of the electrode array after the insertion of the electrode array has completed, and
- Fig. 13: illustrates the system.

### DETAILED DESCRIPTION

The detailed description set forth below in connection with the appended drawings is intended as a description of various configurations. The detailed description includes specific details for the purpose of providing a thorough understanding of various concepts. However, it will be apparent to those skilled in the art that these concepts may be practiced without these specific details. Several aspects of the apparatus and methods are described by various blocks, functional units, modules, components, etc. (collectively referred to as "elements"). Depending upon particular application, design constraints or other reasons, these elements may be implemented using other equivalent elements.

The hearing aid that is adapted to improve or augment the hearing capability of a user by receiving an acoustic signal from a user's surroundings, generating a corresponding audio signal, possibly modifying the audio signal and providing the possibly modified audio signal as an audible signal to at least one of the user's ears. Such audible signals may be provided in the form of an acoustic signal transferred as mechanical vibrations to the user's inner ears through bone structure of the user's head.

The hearing aid is adapted to be worn in any known way. This may include arranging a unit of the hearing aid attached to a fixture implanted into the skull bone such as in a Bone Anchored Hearing Aid or at least a part of the hearing aid may be an implanted part.

A "hearing system" refers to a system comprising one or two hearing aids, and a "binaural hearing system" refers to a system comprising two hearing aids where the devices are adapted to cooperatively provide audible signals to both of the user's ears or the hearing aid of bone conduction type may be part of a bimodal system comprising a cochlea implant and a bone conduction hearing aid. The system may further include auxiliary device(s) that communicates with at least one hearing aid, the auxiliary device affecting the operation of the hearing aids and/or benefitting from the functioning of the hearing aids. A wired or wireless communication link between the at least one hearing aid and the auxiliary device is established that allows for exchanging information (e.g. control and status signals, possibly audio signals) between the at least one hearing aid and the auxiliary device. Such auxiliary devices may include at least one of remote controls, remote microphones, audio gateway devices, mobile phones, public-address systems, car audio systems or music players or a combination thereof. The audio gateway is adapted to receive a multitude of audio signals such as from an entertainment device like a TV or a music player, a telephone apparatus like a mobile telephone or a computer, a PC. The audio gateway is further adapted to select and/or combine an appropriate one of the received audio signals (or combination of signals) for transmission to the at least one hearing aid. The remote control is adapted to control functionality and operation of the at least one hearing aids. The function of the remote control may be implemented in a SmartPhone or other electronic device, the SmartPhone/ electronic device possibly running an application that controls functionality of the at least one hearing aid.

In general, a hearing aid includes i) an input unit such as a microphone for receiving an acoustic signal from a user's surroundings and providing a corresponding input audio signal, and/or ii) a receiving unit for electronically receiving an input audio signal. The hearing aid further includes a signal processing unit for processing the input audio signal and an output unit for providing an audible signal to the user in dependence on the processed audio signal.

The input unit may include multiple input microphones, e.g. for providing direction-dependent audio signal processing. Such directional microphone system is adapted to enhance a target acoustic source among a multitude of acoustic sources in the user's environment. In one aspect, the directional system is adapted to detect (such as adaptively detect) from which direction a particular part of the microphone signal originates. This may be achieved by using conventionally known methods. The signal processing unit may include amplifier that is adapted to apply a frequency dependent gain to the input audio signal. The signal processing unit may further be adapted to provide other relevant functionality such as compression, noise reduction, etc. The output unit may include an output transducer for providing mechanical vibrations either transcutaneously or percutaneously to the skull bone.

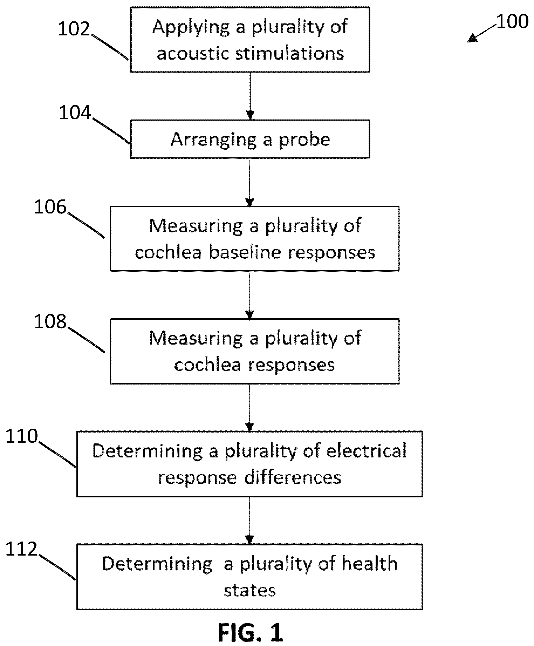

Referring to Fig. 1, a diagram explaining a method 100 for detecting health conditions of a cochlea 2 during insertion of an electrode array 4 of a cochlea implant system 6 is seen. The method 100 comprising applying 102 a plurality of acoustic stimulations 8, and where each of the plurality of acoustic stimulations 8 includes a tone burst with different frequencies. An electrocochleography probe 10 is arranged 104 to an outer wall of the recipient's cochlea, and measuring 106 via the electrocochleography probe 10 a plurality of cochlea baseline responses 12 based on the plurality of acoustic stimulations 8, before insertion of the electrode array 4 into the recipient's cochlea 2. Then, a plurality of cochlea responses 11 is measured 108 continuously via the electrocochleography probe 10 based on the plurality of acoustic stimulations 8, during insertion of the electrode array 4 into the recipient's cochlea 2. A plurality of electrical response differences 13 between the plurality of cochlea baseline responses 12 and the plurality of cochlea responses 11, respectively, is determined 110, and a plurality of health conditions 15 of the cochlea 2 is determined 112 during insertion of the electrode array 4 based on the plurality of electrical response differences 13.

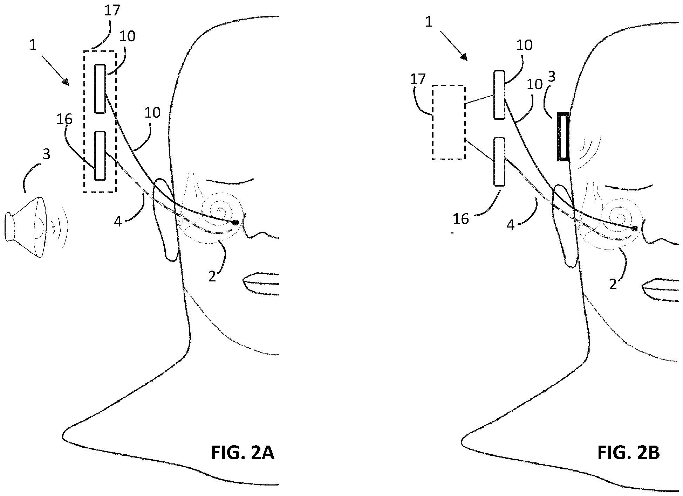

Figs. 2A and 2B illustrate different configuration of a system 1 for performing the method 100. Both figures illustrate a system 1 which includes a receiver 3 for applying a plurality of acoustic stimulations to a recipient. In Fig. 2A, the receiver 3 is a speaker and in Fig. 2B the receiver 3 is a bone conduction device. The system 1 includes an electrode array 4 which is connected to a stimulator processor 16. The stimulator processor 16 is configured to apply the electrical stimulation to each of the electrodes of the electrode array 4.In Fig. 2A, the stimulator processor 16 is part of the processor unit 17, and in Fig. 2B, the stimulator processor 16 is connected externally to the processor unit 17. Furthermore, the system 1 includes an electrocochleagraphy probe 10 which is arranged on to an outer wall of the recipient's cochlea 2. In Fig. 2A, the probe 10 is part of the processor unit 17, and in Fig. 2B the probe 10 is connected externally to the processor unit 17. The probe 10 is configured to perform measurements of a plurality of cochlea baseline responses based on the plurality of acoustic stimulations, before insertion of the electrode array into the recipient's cochlea, and to perform continuously or sequentially measurements of a plurality of cochlea responses based on the plurality of acoustic stimulations, during insertion of the electrode array into the recipient's cochlea.

The stimulator processor 16 is not essential for the system to perform the method 100.

The processor unit 17 is configured to determine a plurality of electrical response differences between the plurality of cochlea baseline responses and the plurality of cochlea responses, received from the probe 10. Based on the plurality of electrical response differences a plurality of health conditions at different location of the cochlea is determined during insertion of the electrode array.

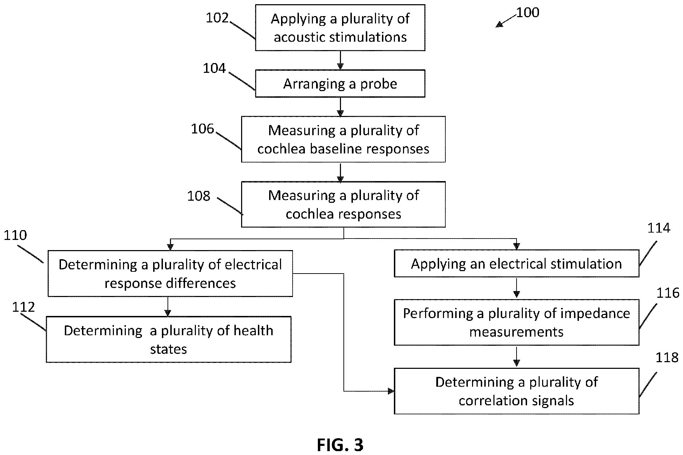

Fig. 3 illustrates a diagram of the method 100 which further comprises applying 114 sequentially an electrical stimulation to each of a plurality of electrodes of the electrode array 4, performing 116 a plurality of impedance measurements of each plurality of electrodes during insertion of the electrode array 4 into the recipient's cochlea 2 based on the electrical stimulations, and determining 118 a plurality of correlation signals based on the plurality of impedance measurements and the plurality of electrical response differences, respectively.

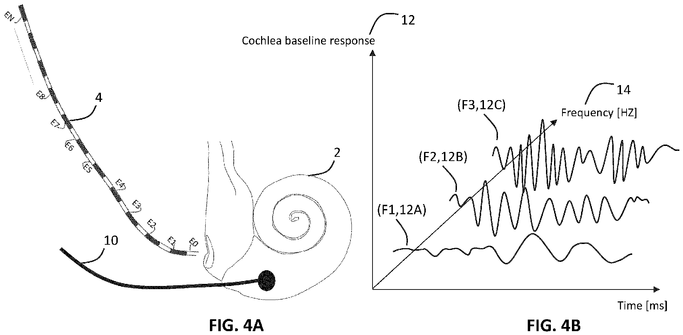

Figs. 4A to 4F illustrate the method 100 for detecting a plurality of health conditions of the cochlea 2 during insertion of the electrode array 4. The electrode array 4 includes N number of electrodes (E0 to EN) distributed along a longitudinal axis of the electrode array 4. Th electrocochleography probe 10 is applied on to the outer wall of the cochlea 2 for measuring the cochlea response (11,12) of the cochlea 2 while applying a plurality of acoustic stimulations 8 which includes a tone burst. In the examples illustrated in Figs. 4A to 4F, three tone bursts with three different frequencies (F1, F2, F3) are used. In Figs. 4A and 4B, a cochlea baseline response (12A, 12B, 12C) is measured for each of the tone burst over a time period while not inserting the electrode array 4 into the cochlea 2.

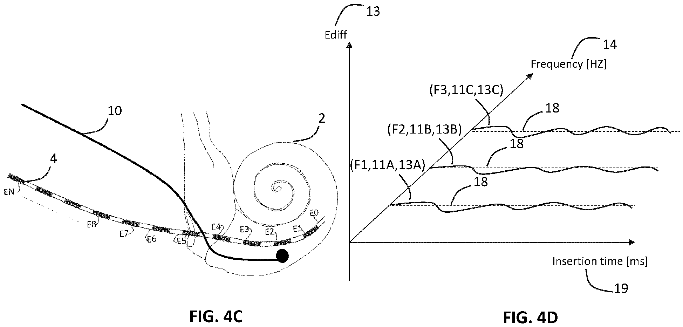

In Figs. 4C and 4D, the electrode array 4 is inserted into the cochlea 2 while applying the plurality of acoustic stimulations 8 including the three frequencies (F1, F2, F3). A cochlea response (11A,11B,11C) is measured for each of the acoustic stimulation 8 while inserting the electrode array 4, and an electrical response difference (13A,13B,13C) is determined by determining the difference between the measured cochlea response (11A,11B,11C) and the respective cochlea baseline response (12A, 12B, 12C) which is measured while applying an acoustic stimulation 8 having a tone burst with the same frequency. The electrical response difference (13A,13B,13C) is determined as a function of an insertion time 19.

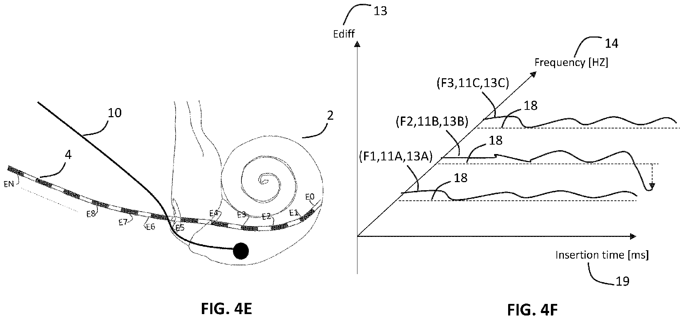

In Figs. 4E and 4F, the electrode array 4 is inserted into the cochlea 2 while applying the plurality of acoustic stimulations 8, but in this example, the electrode array 4 collides with the inner wall of the cochlea 2. The collision between the electrode array 4 and the inner wall is depicted in Fig. 4F as a drop in the determined electrical response difference 13B while applying an acoustic stimulation 8 which includes frequency F2. The drop in the electrical response difference 13B indicates that the cochlea 2 is in a second health condition, which in this specific example, leads to a lower residual hearing. The method 100 or the system 1 is configured to determine a plurality of health conditions of the cochlea 2 which includes at least a first health condition and at least a second health condition, and where the cochlea 2 is in the at least first health condition if the determined electrical response difference 13 is above or equal a response difference threshold 18, and where the cochlea 2 is in the at least second health condition if the determined electrical response difference 13 is below the response difference threshold 18.

In the example illustrated in Fig. 4F, the drop in the electrical response difference 13B in response to a tone burst with frequency F2 represents a collision between the electrode array 4 and an area of the inner wall of the cochlea 2 which includes hair cells tuned to a frequency range including frequency F2.

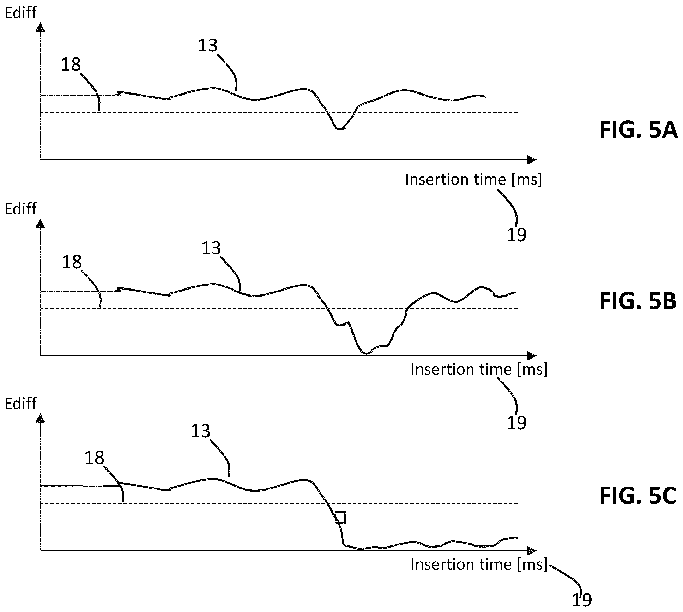

Figs.5A to 5C illustrate different examples of a situation where the electrode array 4 collides with the inner wall of the cochlea 2. In Fig. 5A, the determined electrical response difference 13 drops below the response difference threshold 18 and increases again after a short period of time above the threshold 18. In this situation the health condition of the cochlea is a temporal lowering of the cochlea response 11 over a short period of time which may lead to a slight lowering of the residual hearing of the cochlea.

In Fig. 5B, the determined electrical response difference 13 drops below the response difference threshold 18 and increases again above the threshold 18 after a longer period than in the example illustrated in Fig. 5A. In this situation, the health condition of the cochlea is a temporal lowering of the cochlea response 11 over a long period of time which may lead to a more severe lowering of the residual hearing of the cochlea when comparing to the example where the temporal lowering of the cochlea response 11 is over a short period of time.

In Fig. 5C, the determined electrical response difference 13 drops fast below the response difference threshold 18 and does not increase again above the threshold 18. In this situation, the health condition of the cochlea is a permanently lowering of the cochlea response 11 which leads to a permanently lowering of the residual hearing of the cochlea 2.

The measured cochlea response 11 and the determined electrical response difference 13 may be performed continuously while inserting the electrode array 4 into the cochlea 2, and the determined electrical response difference 13 may be visualized to a surgency through a graphical user interface. The graphical user interface may receive data from the processing unit 17 and/or the probe 19, where the data includes measured cochlea response 11, measured cochlea baseline response 12 and/or determined electrical response difference 13. The data is continuously updated. The graphical user interface helps the surgency to immediately stop the insertion when seeing a drop in the electrical response difference 13. An immediately reaction of the surgency will lead to a reduction in the lowering of the residual hearing because a further damaging of the residual hearing is avoided.

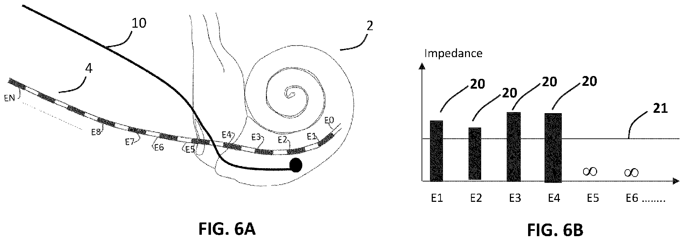

Figs. 6A and 6B illustrate the method 100 and the system configured to performing a plurality of impedance measurements. A stimulator processor 16 is connected to the electrode array 4 and applies sequentially an electrical stimulation to each of the plurality of electrodes (E0 to EN) of the electrode array 4. For each of the electrodes (E0 to EN) an impedance measurement 20 is perform while inserting the electrode array 4 into the cochlea 2. The impedance measurement 20 is perform via the electrode (E0 to EN) and the processor unit 17. In Fig. 6A, electrodes E0 to E4 are within the cochlea and the remaining electrodes are outside the cochlea, and in Fig. 6B an impedance is measurable for electrodes E0 to E4 and for the remaining electrodes the impedance is not measurable. Thereby, the processor unit 17 is configured to determine whether an electrode (E0 to EN) of the electrode array 4 is in a first or in a second state. An electrode (E0 to EN) is in the first state if the measured impedance 20 is above a first impedance level 21, and the electrode (E0 to EN) is in the second state if the measured impedance 20 is below the first impedance level 21. When the electrode (E0 to EN) is in the first state, the electrode is within the cochlea, and when the electrode (E0 to EN) is in the second state, the electrode is outside the cochlea.

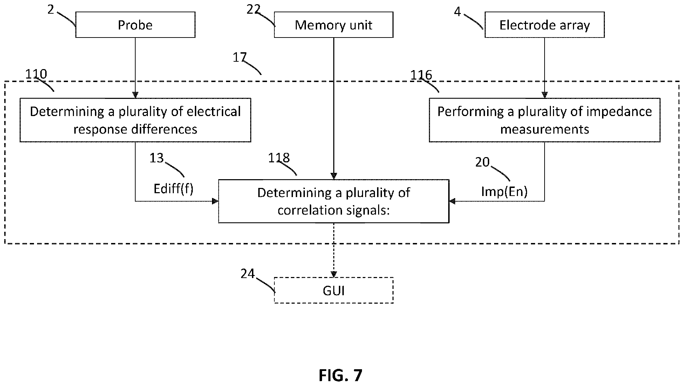

Fig. 7 illustrates in more detail how the method 100 and the system 1 determines a plurality of correlation signals, wherein the plurality of correlation signals includes information about why a drop of the electrical response difference 13 is seen. The processor unit 17 receives from the electrocochleography probe 10 a plurality of cochlea responses 11 and a baseline cochlea response 12 from which the processor unit 17 is configured to determine a plurality of electrical response differences 13 for each of the plurality of acoustic stimulations 8. Furthermore, the processor unit receives from a memory unit 22 at least a length of the electrode array 4 and/or a distance between each of the electrodes (E0 to EN) of the electrode array 4. Additionally, the processor unit 17 receives an output from each of the electrodes (E0 to EN) of the electrode array 4 which is used for measuring the impedance 20 for each of the electrodes (E0 to EN). The measured impedance 20 for each of the electrodes (E0 to EN) and the plurality of electrical response differences 13 are used for determine a plurality of correlation signals 118. The plurality of correlation signals is transferred to a graphical user interface 24.

For example, a drop in the electrical response difference 13 for a given tone burst which includes frequency F is detected, and the drop represents a potential collision between the electrode array 4 and at an area of the inner wall of the cochlea 2 which includes hair cells tuned to a frequency range including frequency F. A first position of the collision within the cochlea is then determined by extracting the position from the physiological cochlea place-frequency map based on the frequency F. Then a second position of for example the most apical electrode E0 of the plurality of electrodes of the electrode array 4 is determined. The second position of the most apical electrode E0 is then determined based on the plurality of impedance measurements 20 and the distance between each of the plurality of electrodes. For example, lets assume that five electrodes (E0 to EN) have a measurable impedance, that means, the five electrodes (E0 to EN) are within the cochlea 2. The distance between each of the plurality of electrodes is constant 2 mm. The length of the electrode array 4 which is within the cochlea 2 may be determined to be 10 mm (5 times 2 mm). Then, the second position of the most apical electrode is then determined by extracting the position from the physiological cochlea place-frequency map based on the length of the electrode array 4 which is within the cochlea.

For example, the first position of the collision is greater than the second position of the most apical electrode E0, has the meaning that the most apical electrode E0 of the electrode array 4 has not yet passed the first position. Thereby, the correlated signal includes information about that the probe 10 has an error.

In another example, the first position of the collision is equal than the second position of the most apical electrode, has the meaning that the most apical electrode of the electrode array 4 is located at the collision point. Thereby, the correlated signal includes information about that the most apical electrode E0 has collided with the cochlea 2.

In yet another example, the first position of the collision is less than the second position of the most apical electrode, has the meaning that the most apical electrode of the electrode array 4 has passed the collision point. Thereby, the correlated signal includes information about that a part of the electrode array 4 (not including the most apical electrode E0) has collided with the cochlea 2.

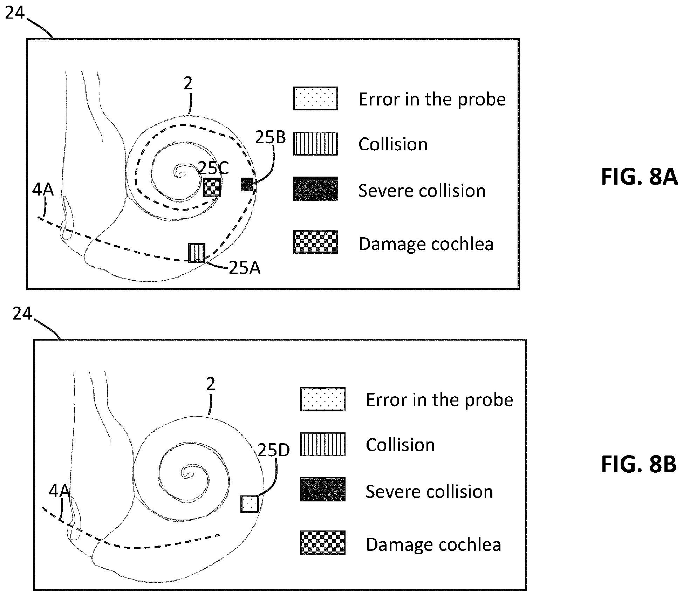

Figs. 8A and 8B illustrate a graphical user interface which shows the information of the correlated signal (25A, 25B, 25C, 25D) on a drawing of a cochlea 2. Furthermore, the graphical user interface 24 includes an estimated position of each of the electrodes of the electrode array 4 which are inside the cochlea 2. In Fig. 8A, the electrode array 4 is fully inserted into the cochlea 2, and three collisions between the electrode array 4 and the cochlea have been detected by the system 1. The position and the content of correlated signal at that given position is marked with a marker (25A,25B, 25C). In Fig. 8B, the electrode array 4 is only partly inserted into the cochlea 2. A collision is detected but the marker 25D tells that an error in the probe has appeared.

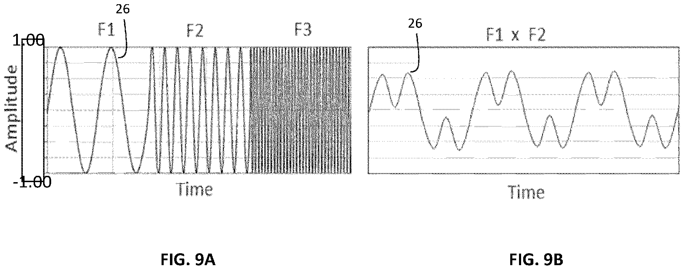

Figs. 9A and 9B illustrate different examples of a plurality of acoustic stimulations 8 including a tone burst. Fig. 9A illustrates a tone burst which comprises a sweep tone that has a continuous acoustic signal in which the frequency varies over time. In this specific example, the tone burst includes frequencies F1, F2 and F3. Fig. 9B illustrate a tone burst with a harmonic complex acoustic signal which includes a convolution or a multiplication of different tones. In this example, the tone burst includes a mix of frequencies F1 and F2.

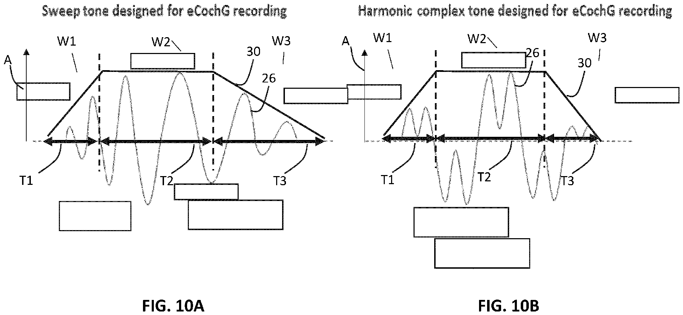

Figs. 10A and 10B illustrate that the tone burst 26 is shaped by an electrocochleography profile 30. The profile 30 is configured to adapt the shape of the tone burst 26 differently in different time windows (W1, W2, W3), and each of the time windows (W1, W2, W3) has a given length (T1, T2, T3). In Fig. 10A the tone burst 26 includes the sweep tone, and in this specific example, the tone burst includes frequencies F1 and F2. In a first time window W1, the profile 30 has an increasing profile which adapts the amplitude A of the tone burst 26 to have an increasing amplitude. In a second window W2 the profile 30 has a constant profile which adapts the amplitude A of the tone burst 26 to have a constant amplitude. In a third window W3 the profile 30 has a decreasing profile which adapts the amplitude A of the tone burst 26 to have a decreasing amplitude. Fig. 10B illustrates a similar example for the tone burst 26 including the harmonic complex acoustic signal. In this specific examples the tone burst 26 includes frequencies F1 and F2.

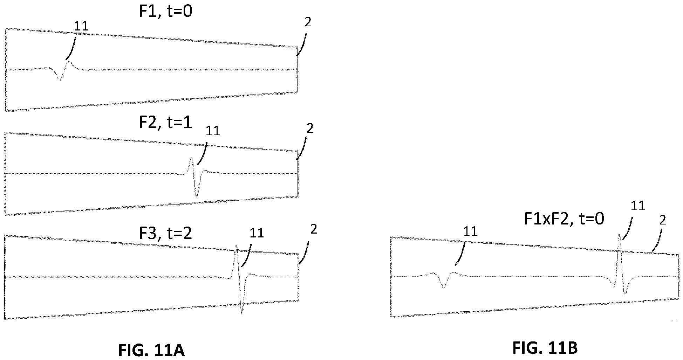

Fig. 11A illustrates a position of a cochlea response within a cochlea 2 based on an acoustic stimulation 8 which includes a sweep tone. At time, t=0, the cochlea response 11 is measured for frequency F1, at time, t=1, the cochlea response 11 is measured for frequency F2, and at time t=2, the cochlea response 11 is measured for frequency F3. In this example, each of the plurality of electrical response differences 13 is determined continuously, and the fastness of detecting a potential collision between the electrode array 4 and the cochlea 2 is depending on the sweep time from the start frequency to the end frequency, which in this example is from frequency F1 to frequency F3.

Fig. 11B illustrates a position of a cochlea response within a cochlea 2 based on an acoustic stimulation 8 which includes a harmonic complex acoustic signal. At time, t=0, the cochlea response 11 is measured for frequency F1 and frequency F2, and in this example, each of the plurality of electrical response differences 13 for frequency F1 and F2 are determined at the same. In this example, the fastness of detecting a potential collision between the electrode array 4 and the cochlea 2 is independent on the sweep time from the start frequency to the end frequency. Therefore, the advantage of using a tone burst which includes a harmonic complex acoustic signal is that a potential collision is faster detected when comparing to a tone burst which includes a sweep tone.

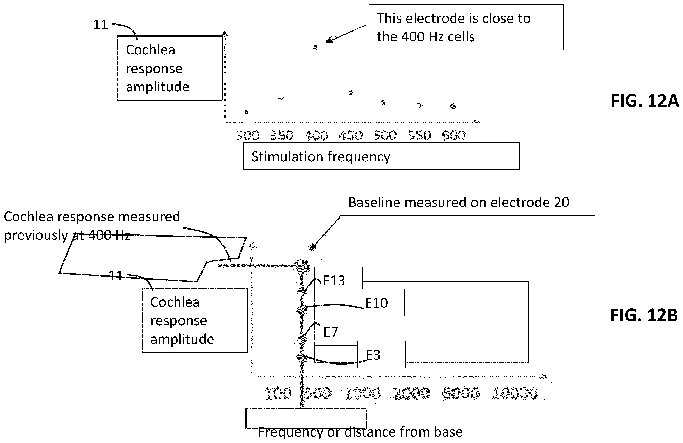

Figs. 12A to 12D illustrate an example of determining a frequency map of the electrodes of the electrode array 4 after the insertion of the electrode array 4 has completed. In Fig. 12A, a baseline frequency and a baseline cochlea response of a given electrode of the electrode array 4 is found. This is achieved by applying a plurality of acoustic stimulations 8 to the user of a cochlea implant system 6 including the inserted electrode array 4, and where each of the plurality of acoustic stimulations 8 includes a tone burst 26 with different frequencies, such as 300 Hz, 350 Hz, 400 Hz, 450 Hz, 500 Hz, 550 Hz, and 600 Hz. Then, a cochlea response 11, i.e. cochlea amplitude response, is measured by one or more of the electrodes of the electrode array and for each of the different frequencies. For example, the frequency of the different frequencies which provides the highest cochlea response on any of the electrodes (E0 to EN), i.e. the highest eCOchG amplitude response, is the baseline frequency, and the highest cochlea response is the baseline cochlea response. The one electrode is preferably the most apical electrode of the electrodes of the electrode array. For example, the baseline frequency response is 400 Hz and the one electrode is the most apical electrode, i.e. electrode number 20.

The baseline frequency and the baseline cochlea response for the one electrode may be stored in the memory unit of the system or within a memory of the cochlea implant system.

The one electrode is denoted as the baseline electrode.

In Fig. 12B, a single acoustic stimulation 8 is applied to the user, wherein the single acoustic stimulation 8 includes the baseline frequency, e.g. 400 Hz. A cochlea response 11 is measured by a group of electrodes (E3, E7, E10,E13) which includes electrodes of the electrode array 4 other than the baseline electrode, e.g. other than electrode number 20.

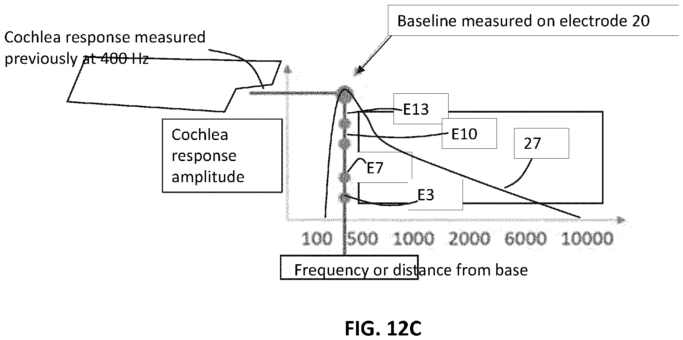

In Fig.12C, a cochlea response model 27 including a cochlea response distribution along a cochlea 2 to a given tone may be derived based on collected data, a general shape of a cochlea response distribution profile along a cochlea, surface image or 3D field image of the cochlea. The cochlea response distribution of the cochlea response model 27 includes a maxima cochlea response 28, and the maxima cochlea response 28 is centered at the baseline frequency, and the maxima cochlea response is equal to the baseline cochlea response.

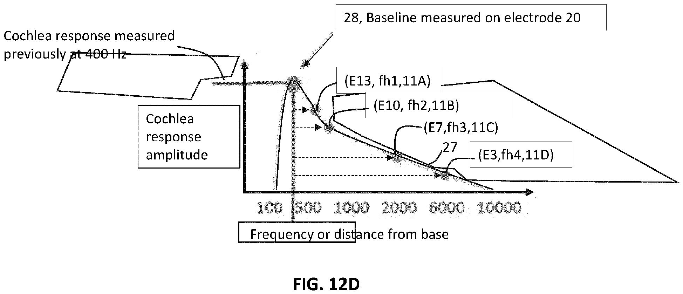

In Fig. 12 D, the measured cochlea response 11 for each of the electrode of the group of electrodes (E3, E7, E10, E13) is matched to a hypothesized frequency (th1,th2,th3,fh4) where the measured cochlea response 11 is equal to a theoretical cochlea response (11A,11B,11C,11D) of the cochlea response model 27.

Each of the electrode of the group of electrodes (E3,E7,E10,E13) is matched to a hypothesized frequency (fh1,fh2,fh3,fh4).

Thereby, the frequency map of the electrode array 4 may include the baseline frequency of the baseline electrode and the hypothesized frequencies (fh1,fh2,fh3,fh4) of the group of electrodes (E3,E7,E10,E13).

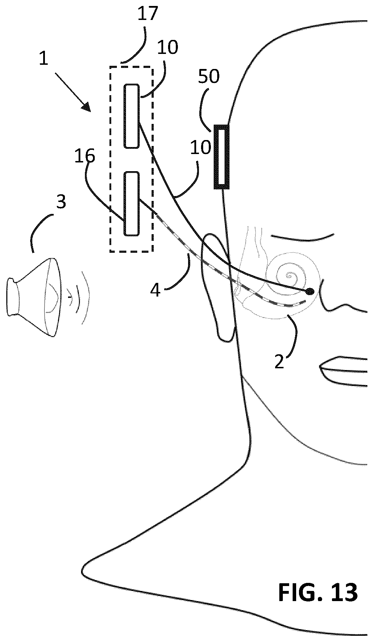

The frequency map of the electrode array 4 may be derived by applying a curve fitting method to the baseline frequency of the baseline electrode and the hypothesized frequencies (fh1,fh2,fh3,fh4) of the group of electrodes (E3,E7,E10,E13) for determine a hypothesized frequency (fh1,fh2,fh3,fh4) to each of the electrodes of the electrode array 4 or the remaining electrodes of the electrode array 4. Fig. 13 illustrates the system 1 which includes a vibrator 50 configured to evokes acoustic soundwaves into the skull of the recipient, and the acoustic soundwaves will reduce the friction force while inserting the electrode array 4. For the acoustic soundwave not to interfere with the measurements of the cochlea responses, the frequencies(y) of the acoustic soundwaves are selected to be different from the frequency of the tone burst.

As used, the singular forms "a," "an," and "the" are intended to include the plural forms as well (i.e. to have the meaning "at least one"), unless expressly stated otherwise. It will be further understood that the terms "includes," "comprises," "including," and/or "comprising," when used in this specification, specify the presence of stated features, elements, components, and/or steps but do not preclude the presence or addition of one or more other features, elements, components, and/or steps thereof. It will also be understood that when an element is referred to as being "connected" or "coupled" to another element, it can be directly connected or coupled to the other element, but an intervening element may also be present, unless expressly stated otherwise. As used herein, the term "and/or" includes any and all combinations of one or more of the associated listed items. The steps of any disclosed method are not limited to the exact order stated herein, unless expressly stated otherwise.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art, and the generic principles defined herein may be applied to other aspects.

Further embodiments of the present disclosure can include the following features: Embodiment 1: A method for detecting a plurality of health conditions of a cochlea during insertion of an electrode array of a cochlea implant system into the cochlea of a recipient, wherein the method comprises;
- applying a plurality of acoustic stimulations, and where each of the plurality of acoustic stimulations includes a tone burst including multiple acoustic pulses modulated at a tone of one frequency,
- arranging an electrocochleography probe to an outer wall of the recipient's cochlea,
- measuring via the electrocochleography probe a plurality of cochlea baseline responses based on the plurality of acoustic stimulations, before insertion of the electrode array into the recipient's cochlea,
- measuring continuously via the electrocochleography probe a plurality of cochlea responses based on the plurality of acoustic stimulations, during insertion of the electrode array into the recipient's cochlea,
- determining a plurality of electrical response differences between the plurality of cochlea baseline responses and the plurality of cochlea responses, respectively, and
- determining a plurality of health conditions of the cochlea during insertion of the electrode array based on the plurality of electrical response differences.

Embodiment 2: A method according to embodiment 1, wherein the plurality of baseline responses and the plurality of cochlea responses include measurements of cochlea microphonics of the cochlea.

Embodiment 3: A method according to any of the embodiments, wherein the acoustic stimulation is provided by a receiver configured to provide the plurality of acoustic stimulations into an ear canal of the recipient, or the plurality of acoustic stimulations is provided by a bone conduction device configured to provide the plurality of acoustic stimulations via a temporal bone of the recipient.

Embodiment 4: A method according to any of the embodiments, wherein the electrical array includes a plurality of electrodes, and wherein the method comprises;
• applying sequentially an electrical stimulation to each of a plurality of electrodes of the electrode array via a stimulator processor connected to the electrode array,
• performing a plurality of impedance measurements of each plurality of electrodes during insertion of the electrode array into the recipient's cochlea based on the electrical stimulations. and
• determining a plurality of correlation signals based on the plurality of impedance measurements and the plurality of electrical response differences, respectively.

Embodiment 5: A method according to embodiment 4, where the plurality of correlation signals is further determined based on a length of the electrode array, a length of each of the plurality of electrodes, and/or a distance between each of the plurality of electrodes.

Embodiment 6: A method according to any of embodiments 4 and 5, wherein the plurality of impedance measurements comprises determining real-time impedance measurements of each of the plurality of electrodes.

Embodiment 7: A method according to any of embodiments 4 to 6, including determining a first state and a second state for each electrode of the plurality of electrodes based on each of the plurality of impedance measurements, and wherein an electrode of the electrode array is in the first state if the impedance measurement is above a first impedance level, and the electrode is in the second state if the impedance measurement is below the first impedance level.

Embodiment 8: A method according to embodiment 7, wherein the electrode of the electrode array is located outside the cochlea if the electrode is in the first state, and the electrode of the plurality of electrodes is located within the cochlea if the electrode is in the second state.

Embodiment 9: A method according to any of the previous embodiments, wherein the plurality of health conditions includes at least a first health condition and at least a second health condition, and where the cochlea is in the at least first health condition if an electrical response difference of the plurality of electrical response difference is above or equal a response difference threshold, and where the cochlea is in the at least second health condition if the respective electrical response difference of the plurality of electrical response difference is below the response difference threshold.

Embodiment 10: A method according to any of the previous embodiments, wherein the determining of the plurality of health conditions comprises determining real-time plurality of health conditions.

Embodiment 11: A system comprising:
- a receiver configured to apply a plurality of acoustic stimulations to a recipient, and where each of the plurality of acoustic stimulations includes a tone burst including multiple acoustic pulses modulated at a tone of one frequency,
- a cochlea implant system comprising;
   o an electrode array configured to provide an electrical stimulation to auditory nerves of the recipient's cochlea, and the electrode array includes a plurality of electrodes,
- an electrocochleography probe arranged on to an outer wall of the recipient's cochlea, and where the electrocochleography is configured to:
   o perform measurements of a plurality of cochlea baseline responses based on the plurality of acoustic stimulations, before insertion of the electrode array into the recipient's cochlea,
   o perform continuously measurements of a plurality of cochlea responses based on the plurality of acoustic stimulations, during insertion of the electrode array into the recipient's cochlea,
- a processor unit connected at least to the electrocochleography probe, and where the processor is configured to determine a plurality of electrical response differences between the plurality of cochlea baseline responses and the plurality of cochlea responses, respectively, and to determine a plurality of health conditions within the cochlea at different location during insertion of the electrode array based on the plurality of electrical response differences.

Embodiment 12: A system according to embodiment 11, wherein the stimulator processor is configured to:
- apply sequentially an electrical stimulation to each of the plurality of electrodes of the electrical array,
- perform a plurality of impedance measurements of each plurality of electrodes during insertion of the electrode array into the recipient's cochlea based on the electrical stimulations, and
- determine a plurality of correlation signals based on the plurality of impedance measurements and the plurality of electrical response differences, respectively.

Embodiment 13: A system according to embodiment 12, wherein a first state and a second state for each of the electrodes of the plurality of electrodes are determined based on each of the plurality of impedance measurements, and wherein an electrode of the plurality of electrodes is in the first state if the impedance measurement is above a first impedance level, and the electrode is in the second state if the impedance measurement is below the first impedance level.

Embodiment 14: A system according to any of embodiments 11 to 13, includes a graphical user interface, where the graphical user interface is configured to provide a graphical representation of the plurality of electrical response differences as a function of insertion time, physiological cochlea place-frequencies, and/or insertion depths, and where the graphical user interface is configured to receive the alarm signal and create a warning to a user of the graphical user interface.

Embodiment 15: A system according to any of embodiments 11 - 13, includes a graphical user interface, where the graphical user interface is configured to provide a graphical representation of the plurality of correlation signals as a function of physiological cochlea place-frequencies and/or insertion depths, and where the graphical user interface is configured to receive the alarm signal and create a warning to a user of the graphical user interface.

## Claims

1. A system (1), comprising:
a transducer (3) configured to apply acoustic stimulation to a recipient of a cochlear implant;
an electrode array (4) that is configured for insertion into at least a portion of a recipient's cochlea; and
a processor unit (17) coupled to the electrode array (4), wherein
the system is configured to provide a personalized and automatic fitting of a cochlea implant frequency allocation map based on based on eCOchG amplitude responses measured by one or more of the electrodes of the electrode array (4).

2. The system of claim 1, wherein the system is configured to:
apply a plurality of acoustic stimulation with different frequencies to the recipient of the cochlear implant, and
the processor unit (17) is configured to:
measure eCOchG amplitude response by one or more of the electrodes of the electrode array for each of the different frequencies; and
determine a frequency map.

3. The system of claim 2, wherein the processor unit is connected to the electrode array via the sound processor of the cochlear implant.

4. The system of claim 2, wherein the processor unit is connected to the electrode array via an implant of the cochlear implant.

5. The system of one of the claims 1-4, wherein measuring eCOchG amplitude responses to the plurality of acoustic stimulation comprises:
recording the one or more acoustically-evoked responses via one or more of the electrodes of the electrode array.

6. The system of one of the claims 1-5, wherein each of the plurality of acoustic stimulations includes a tone burst with different frequencies.

7. The system of claims 6, wherein the different frequencies comprise 300 Hz, 350 Hz, 400 Hz, 450 Hz, 500 Hz, 550 Hz, and 600 Hz.

8. The system of one of the claims 1-7, wherein the system is configured to find a baseline frequency and a baseline cochlea response of a given electrode of the electrode array.

9. The system of claim 8, wherein the processor unit (17) is configured to, after measurement of eCOchG amplitude responses by one or more of the electrodes of the electrode array for each of the different frequencies, determining the frequency of the different frequencies which provides the highest eCochG amplitude response as the baseline frequency for the given electrode, wherein the given electrode is set as the baseline electrode.

10. The system of claim 9, wherein the system is configured to store the baseline frequency for the given electrode in a memory unit of the system or within a memory of the cochlea implant system.

11. The system of one of the claims 8-10, wherein the system is configured to apply a single acoustic stimulation, wherein the single acoustic stimulation includes the baseline frequency.

12. The system of claim 11, wherein the system is further configured to measure a cochlea response of a group of electrodes, which includes electrodes of the electrode array other than the baseline electrode.

13. The system of claim 12, wherein the system is further configured to derive a cochlea response model based on the measured data including at least one of a cochlea response distribution along a cochlea to a given tone based on collected data,
a general shape of a cochlea response distribution profile along a cochlea, surface image or 3D field image of the cochlea.

14. The system of claim 13, wherein the system is further configured to match the measured cochlea response for each electrode of the group of electrodes to a hypothesized frequency where the measured cochlea response is equal to a theoretical cochlea response of the cochlea response model.

15. The system of claim 14, wherein the system is further configured to derive a frequency map of the electrode array by applying a curve fitting method to the baseline frequency of the baseline electrode and the hypothesized frequencies of the group of electrodes for determine a hypothesized frequency to each of the electrodes of the electrode array or the remaining electrodes of the electrode array.
